# EUROPÄISCHE PATENTANMELDUNG

(11) **EP 2 147 637 A1**
(43) Veröffentlichungstag der Anmeldung: **27.01.2010**
(21) Anmeldenummer: 09163494.9
(22) Anmeldetag: 23.06.2009
(51) Int. Cl.: A61B 8/12, B01L 3/00, G01N 33/574, G06F 19/00

(54) **Verfahren und Vorrichtung zur Ermittlung eines eine Prostata eines Patienten betreffenden Parameters**

(30) Priorität: 25.07.2008 DE 102008034703
(71) Anmelder: SIEMENS AKTIENGESELLSCHAFT, 80333 München (DE)
(72) Erfinder: Fehre, Jens, 91353, Hausen (DE); Nanke, Ralf, 91077, Neunkirchen am Brand (DE); Stetter, Martin, 80997, München (DE)

(57) **Zusammenfassung**

Bei einem Verfahren zur Ermittlung wenigstens eines eine Prostata (52) eines Patienten (2) betreffenden Parameters führt ein integriertes Diagnoseunterstützungssystem (30) folgende Schritte durch: Anhand einer Screeningmethode (8) wird ein erster Risikokennwert (12) für Prostataerkrankung ermittelt, falls dieser einen ersten Grenzwert (14) übersteigt, wird anhand eines ein medizinisches Bild (50) des Patienten (2) liefernden Verfahrens (16) wenigstens ein zweiter Risikokennwert (18) für Prostataerkrankung und deren Ort (62) ermittelt. Ein integriertes Reportsystem (38) gibt als Parameter mindestens einen der Risikokennwerte (12,18) und/oder den Ort (62) aus.

Eine entsprechende Vorrichtung (30) umfasst eine Minilabor-Analyseeinheit (34) für den Screening-Test (8), ein intelligentes Decision-Support-System (10) für den ersten Risikokennwert (12), eine ersten Schnittstelle (46) zu einem Bildgebungssystem (48), ein Evaluationssystem (60) für das medizinische Bild (50) für den zweiten Risikokennwert (18), ein Reportsystem (38) mit einer Benutzerschnittstelle (39)

## Beschreibung

Verfahren und Vorrichtung zur Ermittlung eines eine Prostata eines Patienten betreffenden Parameters

Ziel der heutigen Medizin ist es, eine insbesondere komplexe Erkrankung eines Patienten möglichst passgenau und rechtzeitig zu therapieren. Hierzu ist es notwendig, das Vorhandensein und den Typ der Erkrankung möglichst früh und genau zu erkennen. Dies gilt insbesondere für verschiedene Prostataerkrankungen des Mannes einschließlich Prostatakrebs, der beim Mann häufigsten Krebsart.

Bei Prostataerkrankungen soll einerseits eine Früh- und Differenzialdiagnostik bei möglichst vielen potentiell gefährdeten (z.B. älteren) Menschen durchgeführt werden, um die Heilungschancen zu erhöhen und gleichzeitig Kosten von Folgebehandlungen zu reduzieren. Andererseits ist nach der heute üblichen Vorgehensweise die Diagnostik von Prostataerkrankungen ein mehrstufiger und langwieriger Vorgang:

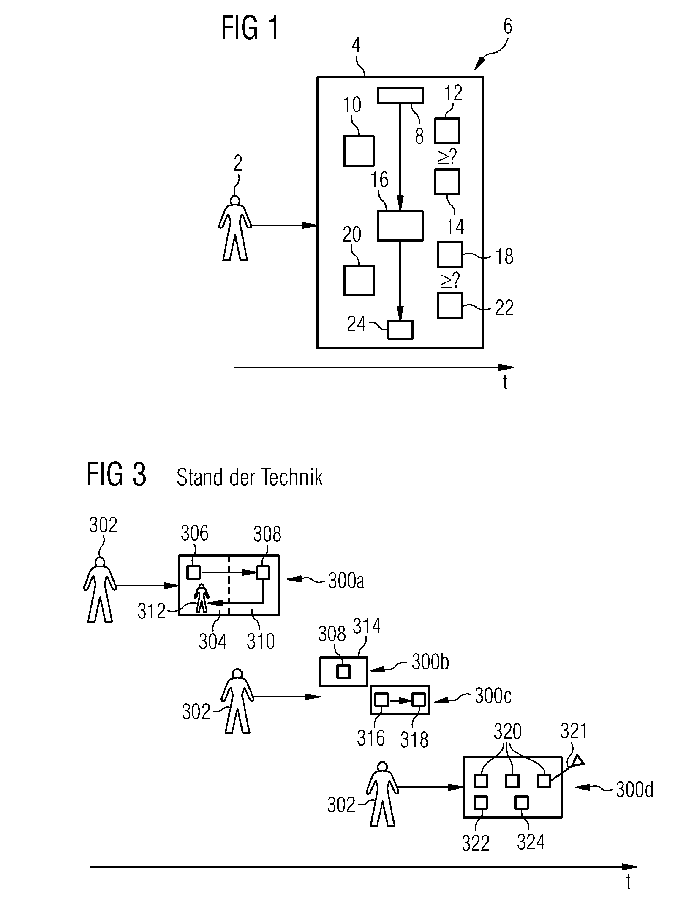

Fig. 3 zeigt in einem Ablaufdiagramm über der Zeit t die heute typische Vorgehensweise bei einer Prostatadiagnostik. Zunächst wird bei einem ersten Besuch 300a eines Patienten 302 in einer Arztpraxis 304 diesem eine Blutprobe 306 entnommen. Zur Bestimmung eines PSA-Wertes 308 - also der Konzentration des "Prostate-Specific Antigen - aus der Blutprobe 306 wird diese in ein Zentrallabor 310 geschickt, das die Auswertung vornimmt und das Ergebnis einem Arzt 312 in der Praxis 304 mitteilt. Der PSA-Wert 308 ist ein Indikator für Prostataerkrankungen, allerdings mit begrenzter Spezifität. Dieser Vorgang erstreckt sich über wenigstens einen, oft mehrere Tage.

Wird diese erste Untersuchung, also der Besuch 300a bei einem niedergelassenen Arzt durchgeführt und zeigt einen Verdacht, wird der Patient 302 an ein Krankenhaus 314 überwiesen, wo bei einem Besuch 300b gemäß o.g. Prozedur der oben genannte PSA-Wert 308 zur Bestätigung üblicherweise nochmals ermittelt wird, wieder durch ein - eventuell anderes - Zentrallabor.

Bestätigt das Ergebnis des PSA-Tests den Verdacht auf eine Prostataerkrankung, so wird der Patient 302 ein zweites Mal zu einem Besuch 300c in das Krankenhaus 314 einbestellt, diesmal zur TRUS- (TransRektalen UltraSchall-) Untersuchung 316. Hierbei wird nach verdachtserhärtenden Momenten lokal an der Prostata 318 des Patienten 302 gesucht. In vielen Fällen wird die TRUS-Untersuchung 316 allerdings auch beim ersten Besuch 300b durchgeführt.

Erhärtet der PSA- oder TRUS-Befund den Krankheitsverdacht, so wird der Patient 302 ein zweites oder drittes Mal zu einem Besuch 300d zur Vornahme einer Biopsie 320 einbestellt. Da nicht sichergestellt werden kann, dass die Biopsienadeln 321 einen eventuellen Krankheitsherd (z.B. eine Entzündung oder einen Tumor) im Patienten 302 auch treffen, muss eine solche Biopsie 320 manchmal auch öfter als einmal durchgeführt werden.

Neuere Ansätze zur Biopsie 320 benutzen eine sogenannte geführte Biopsie, d.h. eine Ultraschallbildgebung 322 mit gleichzeitiger Kontrastmittelgabe 324, um die Biopsienadeln 321 gezielt an den Krankheitsherd zu führen. Dies reduziert die physische und psychische Belastung für den Patienten 302, da in der Regel nur eine einzige Biopsieuntersuchung 320 durchgeführt werden muss.

Insgesamt erstreckt sich damit die in Fig. 3 beschriebene Standardprozedur in der Regel über mehrere Wochen und muss in mehreren Sitzungen bzw. Besuchen 300a-d durchgeführt werden.

Mit dem bekannten Verfahren werden also am Patienten Parameter erhoben bzw. gesammelt. Die Parameter sind z.B. Laborwerte, Ultraschall- oder Durchleuchtungsbilder oder sonstige Befundungswerte. Diese Parameter spiegeln den Zustand der Prostata eines Patienten wieder, stehen also auch im Zusammenhang mit einer Prostataerkrankung des Patienten. Mit anderen Worten bilden solche Parameter die Datenbasis für einen Arzt, der anhand dieser durch seine persönliche Bewertung der Fakten eine Diagnose für den Patienten stellt, um eine Prostataerkrankung auszuschließen oder eben z.B. nach ihrer Art und Schwere zu diagnostizieren.

Aus der am 18.12.2008 offengelegten DE 10 2007 026 910 A1 ist eine medizinische Untersuchungseinheit mit integrierter Minilabor-Analyseeinheit bekannt. Die vor allem in Notfallräumen, Intensivstationen, OP-Räumen etc. einsetzbare Einheit enthält wenigstens ein Ultraschall-, Patientenmonitor-, EKG-, Beatmungs-, und/oder Reanimationsgerät zur Überwachung der Lebensfunktionen und/oder notärztlichen Sofortbehandlung von Notfallpatienten. Anhand dieser Untersuchungseinheit können mehrere medizinische Untersuchungen integriert, d.h. in einem unterbrechungsfreien Arbeitsablauf durchgeführt werden.

Aufgabe der vorliegenden Erfindung ist es, ein verbessertes Verfahren und eine Vorrichtung anzugeben, mit denen die Ermittlung eines o.g., eine Prostata eines Patienten betreffenden Parameters durchgeführt werden kann.

Die Aufgabe wird hinsichtlich des Verfahrens gelöst durch ein Verfahren zur Ermittlung wenigstens eines eine Prostata eines Patienten betreffenden Parameters, mit folgenden Schritten: Die Schritte werden dabei von einer Vorrichtung, d.h. einem integrierten Diagnoseunterstützungssystem in einem integrierten Arbeitsablauf durchgeführt. Zuerst wird am Patienten anhand einer Screeningmethode ein erster Risikokennwert für das Vorhandensein einer Prostataerkrankung ermittelt. Mit anderen Worten wird als Ergebnis des Screening-Tests bzw. als erster Risikokennwert das globale, also körperweit gemessene Risiko für das Vorhandensein einer Prostataerkrankung ermittelt. Im Rahmen des Screenings kann zum Beispiel der etablierte PSA-Level, auch zusammen mit von ihm abgeleiteten Eigenschaften gemessen werden. Auch kann ein Marker-Panel, d.h. mehrere Marker gleichzeitig, mit höherer Sensitivität und Spezifität zum Einsatz kommen.

Falls der erste Risikokennwert einen ersten Grenzwert übersteigt, wird am Patienten anhand eines ein medizinisches Bild, z.B. ein Ultraschall- oder Durchleuchtungsbild, des Patienten liefernden Verfahrens ein zweiter Risikokennwert für das Vorhandensein der Prostataerkrankung und der vermutete Ort der Prostataerkrankung ermittelt.

In diesem Verfahrensschritt wird also mit einer ersten nicht invasiven Methode bei bestehendem globalen Risiko - und nur dann - unter Verwendung von bildgebenden Verfahren wie zum Beispiel Ultraschall als zweiter Risikokennwert das lokale am speziellen Patienten gemessene Risiko für das Bestehen einer Erkrankung aufzeigt. Das Bildgebungsverfahren kann gegebenenfalls kombiniert mit unspezifischen oder krankheitsspezifischen in vivo Markern, z.B. einem Kontrastmittel, durchgeführt werden. Darüber hinaus gibt das durch das bildgebende Verfahren erzeugte medizinische Bild später dem Arzt im Diagnoseschritt näheren Aufschluss über die Art der am Patienten zu erwarteten Erkrankung, z.B. das Vorhandensein von Prostatitis bzw. Prostatakrebs.

Nach mindestens einem der vorgenommenen Schritte gibt ein integriertes Reportsystem als Parameter mindestens einen der im bisherigen Verfahrensablauf ermittelten bzw. bereits zur Verfügung stehenden Werte bzw. Daten, also einen der Risikokennwerte und/oder den Ort aus. Mit anderen Worten werden dem Arzt nach Art eines integrierenden Report-Systems in übersichtlicher, die Enddiagnose unterstützender Weise alle Untersuchungen, Befunde und Risikobewertungen anzeigt. So wird ein Qualitätsstandard der vom Arzt erbrachten Ergebnisse sichergestellt. Es ist außerdem sichergestellt, dass der Verfahrensablauf nach dem Stand der gültigen medizinischen Leitlinien erfolgt.

Die Anzeige eines solchen integrierten Reports erfolgt z.B. über ein User-Interface. Der Report stellt ein zusammenfassendes Summary Sheet des diagnostisch-therapeutischen Verlaufes dar. Er zeigt gemäß medizinischer Leitlinien in qualitätsgesicherter Weise die aktuelle, diagnose- bzw. therapierelevante Daten-, Bild-, Analyse- Befund- und Risikolage an.

Die Erfindung beruht dabei auf der Erkenntnis, geeignete Analyse- und Diagnoseverfahren in einer integrierten, auch durch einen niedergelassenen Arzt durchführbaren Form bereitzustellen. So wird der Vorgang der Parametererhebung, und damit auch die sich daran anschließende Diagnosestellung von Prostataerkrankungen durch einen Arzt beschleunigt und (kosten-)effizienter gemacht. Das Verfahren kann dadurch an einer breiteren Masse zu durchforstender potentiell gefährdeter Menschen durchgeführt werden.

Mit anderen Worten beschreibt das erfindungsgemäße Verfahren eine integrierte Lösung zur Vorbereitung einer Diagnostik und gegebenenfalls Therapie bzw. Theranostik von Prostataerkrankungen. Alle Verfahrensschritte werden durch das Verfahren in einer Art und Weise bereit gestellt, dass sie erstens sofort und zweitens jeweils vor Ort z.B. allesamt in einer Klinik oder bei einem niedergelassenen Arzt vorgenommen werden können.

Wesentliche Merkmale des Verfahrens sind damit die Identifikation sowie die passgenaue Kombination von Komponenten bzw. Einzelschritten zu einem Arbeitsablauf, der einen Arzt, z.B. einen Urologen in optimaler Weise unterstützt.

Das Verfahren bietet einen optimierten Arbeitsablauf für den Urologen an. Dadurch kann der Urologe die Qualität seiner Leistung sichern, die Gesundheitsversorgung seiner Patienten erhöhen, und durch Einsparung unnötiger Diagnoseverfahren die Kosten der Diagnose reduzieren.

Das Verfahren bzw. eine entsprechende Vorrichtung kann modular aufgebaut werden und an die Ansprüche einzelner Krankenhäuser bzw. Praxen angeglichen werden.

Das Verfahren bietet eine große Zeitersparnis der Vorbereitung einer Diagnose im Vergleich zum heutigen Stand. Es vermindert Unannehmlichkeiten und psychische Belastungen beim Patienten. Es lässt sich flexibel auf neue Leitlinien anpassen und effizient optimieren, nämlich durch Ersetzen vorhandener Verfahrensmodule durch jeweils neuere und verbesserte Verfahrensteile. Eine Erweiterung von der reinen Diagnostik z.B. auf eine Therapievorbereitung, z.B. durch integrierte Planung oder Einbindung der Unterstützung von lokalen Therapien, wie z.B. Hochintensivem Fokussiertem Ultraschall (HIFU), ist möglich.

In einer bevorzugten Variante des Verfahrens werden zusätzlich folgende Schritte ausgeführt:
Falls zusätzlich zur o.g. Bedingung für den ersten Grenzwert auch noch der zweite Risikokennwert einen zweiten Grenzwert übersteigt, wird am Patienten anhand eines invasiven Verfahrens am Ort der Prostataerkrankung ein Gewebezustand, also ein lokaler Krankheitszustand ermittelt. Durch das Reportsystem kann dann optional auch der Gewebezustand ausgegeben werden.

In diesem Verfahrensschritt wird also eine zweite, invasive Methode nur bei bestehendem globalen und lokalen Risiko - und nur dann - vorgenommen. Dies erlaubt Parameter am Patienten zu gewinnen, die später bei der Diagnose durch den Arzt erlauben, definitive Klarheit über den Gewebezustand und die Art und Schwere der Erkrankung des Patienten zu gewinnen.

In einer bevorzugten Ausführungsform wird die ScreeningMethode unter Miteinbeziehung eines in vitro Tests durchgeführt. Dieser Test wiederum wird vor Ort in einer Minilabor-Analyseeinheit durchgeführt.

In einer weiteren Ausführungsform wird der erste Risikokennwert unter Verwendung eines IT-basierten Decision Support Systems ermittelt.

In einer alternativen Ausführungsform des Verfahrens wird der zweite Risikokennwert anhand von prostataspezifischen Veränderungen ermittelt. Diese werden dem angefertigten medizinischen Bild entnommen.

In einer Variante des Verfahrens errechnet ein Evaluationssystem, z.B. in Form von Software, wiederum den zweiten Risikokennwert. Auch kann dann die örtliche Verteilung dieses Kennwertes, also des lokalen Risikos, beispielsweise in Form einer Risk Map ermittelt werden.

Die Evaluations-Software kann z.B. zur Auswertung der Resultate von Ultraschall- Untersuchungen vornehmen. Die Software kann z.B. aus kontrastverstärkten Ultraschallbildern die örtliche Wahrscheinlichkeitsverteilung für das Vorhandensein von erkranktem Gewebe, zum Beispiel von Krebsgewebe, ermitteln. Diese Verteilung wird dann als Risk Map bezeichnet.

Bei der Durchführung des invasiven Verfahrens können in einer bevorzugten Ausführungsform die Ergebnisse des bildgebenden Verfahrens, gegebenenfalls überlagert mit der Risk Map, in besonders genauer Weise zur Zielführung von z.B. Biopsienadeln bei der invasiven Methode verwendet werden.

Bei dem Verfahren kann in vorteilhafter Weise die Ermittlung von erstem und zweitem Risikokennwert anhand eines Knowledge-Management-Systems durchgeführt werden. Dieses besteht z.B. aus einer integrierenden medizinischen Patientendatenbank und einem Web-Portal, über das in kontextsensitiver Weise Zusatzinformationen in Relation zur Prostatatheranostik abgerufen werden können.

In einer bevorzugten Ausführungsform des Verfahrens wird eine integrierte medizinische Datenbank eingesetzt, die entweder lokal oder zentral geführt wird. Die Datenlage in der Datenbank erlaubt, die Entscheidungsunterstützung im Verfahrensverlauf, d.h. z.B. die Höhe der Grenzwerte zu optimieren. Die Datenbank kann außerdem zur Qualitätssicherung eingesetzt werden.

In bestimmten Ausgestaltungen bietet das Verfahren damit Software-Unterstützung durch ein Knowledge Discovery- und Knowledge Management System, das die Errechnung quantitativer und damit reproduzierbarer Risikowahrscheinlichkeiten erlaubt.

Hinsichtlich der Vorrichtung wird die Aufgabe gelöst durch eine Vorrichtung zur Ermittlung wenigstens eines eine Prostata eines Patienten betreffenden Parameters. Die Vorrichtung umfasst im Kern z.B. eine Workstation mit verschiedenen Komponenten: Die Vorrichtung umfasst eine an die Workstation angeschlossene Minilabor-Analyseeinheit, zum Beispiel in Form einer Lab-on-a- Chip in vitro Diagnostik-Einheit, zur Messung des PSA-Levels bzw. zusammengesetzter Serum- Marker. Die Minilabor-Analyseeinheit dient damit zur Durchführung eines Screening-Tests am Patienten. Die Vorrichtung umfasst außerdem wenigstens ein intelligentes Decision Support System zur Auswertung der Screening-Daten. Hierbei kann z.B. ein Bayesianisches Netz verwendet werden, dessen Eigenschaften aus Daten über viele Patienten, z.B. durch data mining und knowledge discovery gelernt wurden. Das Decision-Support-System dient damit zur Ermittlung eines ersten Risikokennwertes für das Vorhandensein einer Prostataerkrankung anhand des Screening-Tests.

Die Vorrichtung umfasst eine erste Schnittstelle zu einem Bildgebungssystem zur Anfertigung eines medizinischen Bildes des Patienten, also zu einem Imaging-Gerät. Hier kann z.B. ein im Vergleich zur MR-Untersuchung flexibel einsetzbares Ultraschallgerät verwendet werden.

Zur Vorrichtung gehört noch ein Evaluationssystem zur Ermittlung wenigstens eines zweiten Risikokennwertes für das Vorhandensein der Prostataerkrankung und deren vermuteten Ortes aus dem medizinischen Bild, und ein Reportsystem mit einer Benutzerschnittstelle zur Ausgabe mindestens eines Parameters, wobei der Parameter einer der Risikokennwerte und/oder der Ort ist.

Die erfindungsgemäße Vorrichtung verfügt also über eine Reihe von Schnittstellen. Dies unterstützt einen modularen und daher hochflexiblen Aufbau der Vorrichtung.

Die Vorrichtung und ihre Vorteile wurde ansonsten bereits im Zusammenhang mit dem erfindungsgemäßen Verfahren erläutert. Eine solche Vorrichtung wird z.B. "ProstaStation" genannt und stellt eine integrierte Lösung dar, die z.B. für die Diagnose von Prostatakrebs Verwendung finden kann.

In einer bevorzugten Ausführungsform enthält die Vorrichtung noch eine zweite Schnittstelle zu einem einen Gewebezustand des Patienten am Ort liefernden invasiven Prostatauntersuchungssystem. Durch das Reportsystem kann dann optional auch der Gewebezustand ausgegeben werden.

Wie erwähnt, kann die Vorrichtung eine Schnittstelle zu einem Knowledge-Management-System umfassen, das Parameter liefert, die Prostataerkrankungen betreffen.

Wie ebenfalls erwähnt, kann das Knowledge-Management-System eine medizinische Patientendatenbank und eine Schnittstelle zum Internet umfassen. In diesem Zusammenhang kann dann das Decision Support System dazu benutzt werden, auf der Basis der Datenbankinhalte sowie neuer Patientendaten für einen neuen Patienten ein globales Risiko, also einen ersten Risikokennwert in Form einer Wahrscheinlichkeit zu berechnen.

Für eine weitere Beschreibung der Erfindung wird auf die Ausführungsbeispiele der Zeichnungen verwiesen. Es zeigen, jeweils in einer schematischen Prinzipskizze:
- Fig. 1: den Ablauf einer Parameterermittlung an einem Prostata-Patienten gemäß der Erfindung,
- Fig. 2: den Ablauf aus Fig. 1 im Detail,
- Fig. 3: den Ablauf der Parameterermittlung gemäß Stand der Technik.

Fig. 1 zeigt einen Ablauf einer integrierten Datenerhebung an einem Patienten 2 zum Zweck einer Diagnose einer Prostataerkrankung über der Zeit t in einer generalisierten Darstellung. Die Datenerhebung wird in einer Arztpraxis 4 durchgeführt, der der Patient einen einzigen Besuch 6 abstattet. Als Screeningtest wird zunächst am Patienten ein in-vitro-Test 8 durchgeführt. Ein Decision Support System 10 ermittelt als ersten Risikokennwert ein globales Risiko 12, mit welchem der Patient 2 eine Prostataerkrankung aufweist.

Überschreitet das globale Risiko 12 einen ersten Grenzwert 14, wird am Patienten 2 eine TRUS-Untersuchung 16 durchgeführt. Anhand dieser wird ein lokales Risiko 18 ermittelt, mit welchem der Patient 2 an einer Prostataerkrankung leidet. Gleichzeitig wird über dir TRUS-Untersuchung 16 ein integrierter Befundreport 20 erstellt.

Überschreitet das lokale Risiko 18 einen zweiten Grenzwert 22, wird am Patienten 2 eine Biopsie 24 vorgenommen.

Sämtliche in Fig. 1 gezeigten Schritte werden jeweils sofort aufeinanderfolgend und vor Ort in der Arztpraxis 4 durchgeführt, so dass das gesamte Verfahren lediglich wenige Stunden dauert.

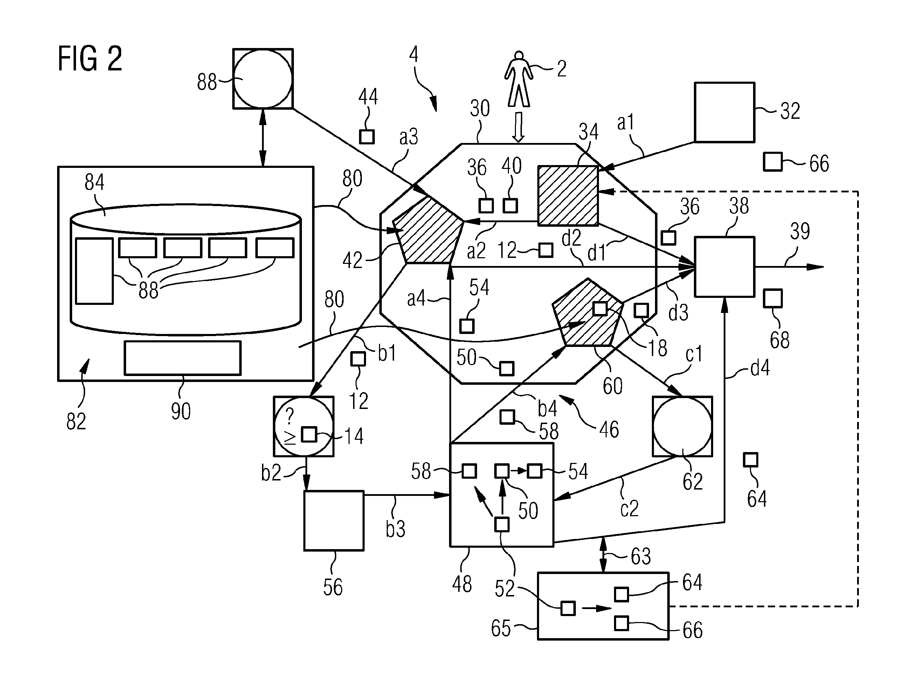

Fig. 2 zeigt den Ablauf des Verfahrens aus Fig. 1 im Detail. Der Patient 2 besucht zur Abklärung von Beschwerden die Arztpraxis 4, die über eine ProstaStation 30 als integrierte Vorrichtung zur Datenerhebung verfügt. Zunächst wird am Patienten ein generell mit a) bezeichnetes Screening durchgeführt. Dem Patienten 2 wird eine Blut und/oder Urin-Probe 32 entnommen und entlang des Pfeils a1 einer zur ProstaStation 30 gehörenden in-vitro-Analyse-Einheit 34 zugeführt.

Die resultierenden Konzentrationswerte 36 werden dann entlang des Pfeils d1 einem Reporting-System 38 zur Anzeige überspielt. Das Reportingsystem verfügt über eine Benutzerschnittstelle 39, an der ein nicht dargestellter Arzt sämtliche Reportergebnisse einsehen kann. Mit d) sind hierbei stets sämtliche Vorgänge bezeichnet, die zu einem integrierten Report gehören. Gleichzeitig werden sie entlang des Pfeils a2 zusammen mit abgeleiteten Größen 40 als Input zum nachgeschalteten Decision-Support-System 10 geleitet. Dieses ist ein Software-System der ProstaStation 30 und führt einen Decision-Support durch. Das Decision-Support-System 10 lädt entlang des Pfeils a3 automatisch weitere verfügbare Daten 44 des Patienten 2, also z.B. Anamnesedaten wie Alter, Gewicht, Historie oder frühere Untersuchungsergebnisse, welche gleichzeitig als Input für eine Risikobewertung dienen.

An der ProstaStation 30 befindet sich eine Schnittstelle 46 zu einem Ultraschallgerät 48. Schließlich werden zur primären Abklärung am Patienten 2 Ultraschallaufnahmen 50 der Prostata 52 des Patienten 2 erstellt. Hieraus werden typische Kennzahlen 54, wie z.B. Prostatavolumen oder das Vorhandensein von Läsionen, ermittelt und entlang des Pfeils a4 dem Decision Support System 10 übermittelt.

Auf der Basis dieser Screeningdaten, z.B. der Konzentrationswerte 36, berechnet das Decision Support System 10 einen Wahrscheinlichkeitswert für das globale Risiko 12, welches dann entlang dem Pfeil d2 dem Reporting System 38 zur Anzeige übermittelt wird.

Überschreitet das globale Risiko entlang des Pfeils b2 den ersten Grenzwert 14 als Schwellwert, bzw. entscheidet optional der Arzt, dass ein Risikofall vorliegt, so erfolgt entlang des Pfeils b2 sofort eine genauere Abklärung der lokalen Verhältnisse in der Prostata 52 in Form einer kontrastmittelverstärkten Ultraschalluntersuchung mit dem Ultraschallgerät 48. Diese erste nicht invasive Untersuchung ist generell mit b) bezeichnet.

Dabei kann zum Beispiel auch ein mit einem molekularen Marker für Prostatakrebs, Prostatitis oder beides versetztes, also molekular markiertes Kontrastmittel 56 entlang des Pfeils b3 injiziert werden. Die resultierenden Kontrastbilder 58 werden entlang des Pfeils b4 einer Analysesoftware 60 zugeführt. Die Analysesoftware 60 als Teil der ProstaStation 30 ermittelt daraus das lokale Risiko 18 in Form einer Risk Map 62 und übermittelt dieses entlang des Pfeils d3 dem Reporting System 38 zur Anzeige.

Überschreiten schließlich auch die lokalen Risikowerte 18 den zweiten Grenzwert 22, bzw. entscheidet der Arzt dass ein erhöhtes Risiko vorliegt, so wird entlang des Pfeils c1 eine zweite invasive Untersuchung c) in Form einer invasiven Gewebeentnahme bzw. Biopsie durchgeführt. Hierzu verfügt die ProstaStation 30 über eine Schnittstelle 63 zu einem invasiven Untersuchungssystem 65. Zur zielgenauen Entnahme im Untersuchungssystem 65 kann hierbei entlang des Pfeils c2 die kontrastmittelverstärkte Ultraschalluntersuchung b dienen, indem die Risk Map 62 benutzt wird.

Im Falle einer Echtzeitberechnung der Risk Map 62 kann insbesondere ein und dieselbe Ultraschalluntersuchung zur ersten Untersuchung b und der Erstellung der Risk Map 62 und zur Unterstützung der zweiten Untersuchung c, also der Biopsie dienen. Dies führt zu einer deutlichen Erhöhung der Effizienz des gesamten Verfahrens und einer deutlichen Reduktion der Belastung für den Patienten 2.

Auch das Ergebnis 64 der Biopsie wird entlang des Pfeils d4 dem Reporting System 38 zugeführt. Dabei kann dieses Ergebnis 64 zum Beispiel von einer klassischen histologischen Untersuchung, z.B. der Bewertung des Gleason Grades stammen. Ein solches Verfahren lässt sich jedoch wegen der erforderlichen Präparationsprozesse nicht in Echtzeit durchführen.

In einem besonders vorteilhaften Ausführungsbeispiel werden daher bei der Biopsie während der Untersuchung c) gewonnenen Gewebeproben 66 gleichfalls der in vitro Analyseeinheit 34 zugeführt, wobei nun nicht Serum-Marker sondern gewebespezifische Krebsmarker gemessen werden. Dies können zum Beispiel Konzentrationswerte von RNA, also Gen-Expression, Proteinen oder Metaboliten sein. Es wird also eine molekulare Diagnostik durchgeführt.

Auf der Basis des nun vollständigen Report Sheets 68 kann dann nach Abschluss des erfindungsgemäßen Verfahrens ein Arzt 70 eine endgültige Diagnose für den Patienten 2 stellen und über den weiteren Behandlungsverlauf entscheiden.

Es ist anzumerken, dass dieses Ausführungsbeispiel sich auf die Diagnostik konzentriert, um die Darstellung übersichtlich zu halten. Im Allgemeinen ist das Konzept der ProstaStation 30 jedoch auch auf Therapieansätze bzw. integrierte Theranostikansätze erweiterbar.

In einer alternativen Ausführungsform der ProstaStation 30 wird entlang der Pfeile 80 die bereits vorhandene Datenlage 82 von ganzen Patientenkohorten zunächst zum Design und später zur Verbesserung bzw. Optimierung des Decision-Support-System 10 und der Analyse-Software 60 herangezogen. Die Datenlage 82 umfasst hierbei eine integrierte medizinische Datenbank 84 in einem Knowledge-Management-System 86, die Patientendaten 88 in Form von Datensätzen enthält. Die Datensätze enthalten z.B. genomische, chemische oder Bilddaten und Laborberichte, Untersuchungsergebnisse usw. Außerdem enthält das Knowledge-Management-System 86 noch ein Web-Portal 90.

So ist es möglich, durch Data Mining, Knowledge Discovery, oder durch andere Verfahren des maschinellen Lernens implizite Zusammenhänge zwischen den Daten aus dem erfindungsgemäßen Verfahren und Befunden aus den kohortenzentrierten Patientendaten der Datenlage 82 zu extrahieren, explizit zu machen und zur verbesserten Analyse zukünftiger Patienten zu verwenden.

In einem anderen Ausführungsbeispiel ist das Knowledge Management System 86 zentral für mehrere ProstaStation-Einheiten 30 gehalten, so dass eine größere und ausgewogenere Datenlage 82 erreicht wird.

## Patentansprüche

1. Verfahren zur Ermittlung wenigstens eines eine Prostata (52) eines Patienten (2) betreffenden Parameters, mit folgenden, von einem integrierten Diagnoseunterstützungssystem (30) in einem integrierten Arbeitsablauf durchgeführten Schritten:
- am Patienten (2) wird anhand einer Screeningmethode (8) ein erster Risikokennwert (12) für das Vorhandensein einer Prostataerkrankung ermittelt,
- falls der erste Risikokennwert (12) einen ersten Grenzwert (14) übersteigt, wird am Patienten (2) anhand eines ein medizinisches Bild (50) des Patienten (2) liefernden Verfahrens (16) wenigstens ein zweiter Risikokennwert (18) für das Vorhandensein der Prostataerkrankung und deren vermuteter Ort (62) ermittelt,
- ein integriertes Reportsystem (38) gibt als Parameter mindestens einen der Risikokennwerte (12,18) und/oder den Ort (62) aus.

2. Verfahren nach Anspruch 1, bei dem zusätzlich folgende Schritte ausgeführt werden:
- falls zusätzlich der zweite Risikokennwert (18) einen zweiten Grenzwert (22) übersteigt, wird am Patienten (2) anhand eines invasiven Verfahrens (24) am Ort (62) der Prostataerkrankung ein Gewebezustand (64) ermittelt,
- das integrierte Reportsystem (38) gibt als Parameter mindestens einen der Risikokennwerte (12,18) und/oder den Ort (62) und/oder den Gewebezustand (64) aus.

3. Verfahren nach Anspruch 1 oder 2, bei dem die Screeningmethode (8) unter Einbeziehung einer vor Ort vorhandenen Minilabor-Analyseeinheit (34) durchgeführt wird.

4. Verfahren nach einem der vorhergehenden Ansprüche, bei dem der erste Risikokennwert (12) unter Verwendung eines IT-basierten Decision-Support-Systems (10) ermittelt wird.

5. Verfahren nach einem der vorhergehenden Ansprüche, bei dem der zweite Risikokennwert (18) anhand prostataspezifischer Veränderungen (54) ermittelt wird.

6. Verfahren nach einem der vorhergehenden Ansprüche, bei dem ein Evaluationssystem (60) als zweiten Risikokennwert (18) eine örtliche Risikoverteilung ermittelt.

7. Verfahren nach einem der vorhergehenden Ansprüche, bei dem das invasive Verfahren (24) das medizinische Bild (50) zur Zielführung nutzt.

8. Verfahren nach einem der vorhergehenden Ansprüche, bei dem die Ermittlung von erstem (12) und zweitem Risikokennwert (18) anhand eines Knowledge-Management-Systems (86) durchgeführt wird.

9. Vorrichtung (30) zur Ermittlung wenigstens eines eine Prostata (52) eines Patienten (2) betreffenden Parameters, mit:
- einer Minilabor-Analyseeinheit (34) zur Durchführung eines Screening-Tests (8) am Patienten (2),
- einem intelligenten Decision-Support-System (10) zur Ermittlung eines ersten Risikokennwertes (12) für das Vorhandensein einer Prostataerkrankung anhand des Screening-Tests (8),
- einer ersten Schnittstelle (46) zu einem Bildgebungssystem (48) zur Anfertigung eines medizinischen Bildes (50) des Patienten (2),
- einem Evaluationssystem (60) zur Ermittlung wenigstens eines zweiten Risikokennwertes (18) für das Vorhandensein der Prostataerkrankung und deren vermuteten Ortes (62) aus dem medizinischen Bild (50),
- einem Reportsystem (38) mit einer Benutzerschnittstelle (39) zur Ausgabe mindestens eines Parameters, wobei dieser einer der Risikokennwerte (12,18) und/oder der Ort (64) ist.

10. Vorrichtung (30) nach Anspruch 9, mit:
- einer zweiten Schnittstelle (63) zu einem einen Gewebezustand (64) des Patienten (2) am Ort (62) liefernden invasiven Prostatauntersuchungssystem (65),
- dem Reportsystem (38) mit der Benutzerschnittstelle (39) zur Ausgabe mindestens eines Parameters, wobei dieser einer der Risikokennwerte (12,18) und/oder der Ort (64) und/oder der Gewebezustand (64) ist.

11. Vorrichtung (30) nach Anspruch 9 oder 10, mit einem Prostataerkrankungen betreffende Parameter (82) liefernden Knowledge-Management-System (86).

12. Vorrichtung nach Anspruch 11, bei dem das Knowledge-Management-System (86) eine medizinische Patientendatenbank (84) und eine Schnittstelle (90) zum Internet umfasst.
